# EUROPEAN PATENT SPECIFICATION

(11) **EP 3 187 227 B1**
(45) Date of publication and mention of the grant of the patent: **11.09.2019**
(21) Application number: 15202794.2
(22) Date of filing: 28.12.2015
(51) Int. Cl.: A61Q 5/00, A61Q 5/12, A61K 8/898

(54) **USE OF SILICONE COPOLYMER FOR IMPROVING SELECT HAIR PROPERTIES AND PROCESS THEREOF**
VERWENDUNG VON SILIKONCOPOLYMER ZUR VERBESSERUNG AUSGEWÄHLTER HAAREIGENSCHAFTEN UND VERFAHREN DAFÜR
UTILISATION DE COPOLYMÈRE DE SILICONE PERMETTANT D'AMÉLIORER LES PROPRIÉTÉS DE CHEVEUX SÉLECTIONNÉS ET PROCÉDÉ ASSOCIÉ

(43) Date of publication of application: 05.07.2017
(73) Proprietor: Kao Corporation, Chuo-ku Tokyo 103-8210 (JP)
(72) Inventor: Burgdorf, Kristin, 64297 Darmstadt (DE); Tate, Yoshimasa, Tokyo, 131-8501 (JP); Schmid, Sabine, 64297 Darmstadt (DE); Cajan, Christine, 64297 Darmstadt (DE)
(74) Representative: Hoffmann Eitle

(56) References cited:
- EP-A1- 2 865 693
- DATABASE GNPD [Online] MINTEL; July 2014 (2014-07), "Hair Styling Lotion for Hair Iron", XP002758381, Database accession no. 2573095

## Description

### Technical Field

The present invention relates to the use of a specific silicone graft copolymer for providing colour retention of artificially coloured hair.

### Background of the Invention

Cosmetic compositions for improving one or more properties of hair have been used daily by the consumers. Conditioners have been provided for improving primarily combability and shine of hair, styling products have been provided for setting hair and especially for increasing volume and body of the hair and increasingly mild surfactant-based cleansing compositions have been provided for eluting less hair colour so that the color remains on the hair for a longer period of time. When a styling product is applied onto the hair and especially, is left on the hair, it usually makes subsequent combing rather difficult. Therefore, products without such disadvantages are very much required.

The silicone copolymer comprised in the compositions of the present invention has been known from the international patent application of Kao Corporation published under the number WO2014/002707 (corresponding to EP 2865693). The application deals solely with the synthesis and certain chemical properties of the polymer. It should be noted that the polymer has primarily been developed for the use in hair setting, styling compositions and there are no disclosure on the advantageous additional effects of the copolymer disclosed in said application.

The inventors of the present invention have unexpectedly found out at the end of a detailed study that the hair treated with the compositions comprising the said copolymer has more shine, less color is eluted from the artificially colored hair when washed subsequently, the polymer friction is reduced and therefore, hair combing becomes easier and the damaging effects of the heat is reduced.

### Summary of the Invention

Accordingly, the first object of the present invention is the use of a composition comprising a silicone graft copolymer comprising an organopolysiloxane segment as a main chain thereof and an unsaturated monomer-derived polymer segment as a side chain thereof,
which is obtainable by firstly reacting an aminopropyl dimethicone with the thiolactone of acetylhomocysteine and then graft copolymerizing the thus obtained mercapto modified dimethicone with a mixture of N,N-dimethylacrylamide and N-t-butylacrylamide, for providing colour retention of artificially coloured hair.

The composition may be used as a leave-on type hair cosmetic. This means that the colour retention can be imparted to the hair in accordance with the present invention by applying the composition comprising the silicone graft copolymer onto hair and allowing the hair to dry without rinsing off.

In order to use the composition for providing colour retention of an artificially coloured hair, the composition may be applied to the hair which has been dyed with a hair color composition. The wash fastness of the hair color is substantially improved. Another aspect of the invention includes a direct dye composition including the above-described silicone graft copolymer and at least one direct dye.

### Detailed Description

In the following, the ingredients and the formulation of the composition will be described in further detail.

### Silicone graft copolymer

The composition of the present invention comprises the silicone graft copolymer as described above. The silicone graft copolymer is a complex copolymer obtained by reacting aminopropyl dimethicone with the thiolactone of acetylhomocysteine and thus mercapto-modified dimethicone is further copolymerized with a mixture of dimethylacrylamide and t-butylacrylamide. Accordingly, the graft copolymer comprises an organopolysiloxane segment as a main chain thereof and an unsaturated monomer-derived polymer segment as a side chain thereof, wherein the main chain is constituted by the mercapto-modified aminopropyl dimethicone while the side chains are constituted of the dimethylacrylamide and t-butylacrylamide moieties.

The synthesis of the said copolymer is made available in the patent publication EP 2865693. The particularly preferred polymer is commercially available from Kao Corporation under the CTFA adopted name Polysilcone-28. The polymer is comprised in the compositions at a concentration in the range of 0.001 to 10%, preferably 0.005 to 7.5%, more preferably 0.01 to 5% and most preferably 0.05 to 3% by weight calculated to the total of the composition.

### Thickening polymer

The composition comprises preferably one or more thickening polymers. The thickening polymer may be selected from anionic, nonionic, cationic and amphoteric ones. With the term thickening polymer it is meant that the polymer increases the viscosity of the solvent used in the compositions. In a preferred embodiment of the present invention the thickening polymer is selected from the polymers having a viscosity of at least 500 mPa·s, preferably 1,000 mPa·s, more preferably 1,500 mPa·s and most preferably 2,000 mPa·s (measured at a polymer concentration of 1% by weight in water at 20°C with a Brookfield viscometer at 10 rpm for 1 minute with an appropriate spindle).

The total concentration of the one or more thickening polymers is in the range of 0.01 to 15%, preferably 0.05 to 10%, more preferable 0.1 to 5%, and most preferably 0.5 to 2 %, by weight calculated to the total of the composition.

### Non-ionic Thickening Polymer

Any cosmetically acceptable non-ionic thickening polymers, which are usually employed in the field of hair cosmetic compositions, may be used as the polymeric thickening agent in the composition of the present invention. The composition may include a single kind or multiple kinds of non-ionic polymer, and may further include one or more anionic, cationic and/or amphoteric polymers in combination with the non-ionic polymer.

Examples for the non-ionic polymers include neutral polysaccharides and derivatives such as ethers or esters thereof. In this respect, neutral gums such as guar gum, hydroxypropyl guar, cellulose ethers such as hydroxyethylcellulose (HEC), methyl hydroxyethylcellulose (MHEC), ethyl hydroxyethylcellulose (EHEC), methyl ethyl hydroxyethylcellulose (MEHEC), hydroxypropylcellulose (HPC), hydroxypropylmethylcellulose (HPMC), hydrophobically modified derivatives thereof such as HM-EHEC, starch and dextrins may be mentioned.

Cellulose ethers such as MHEC or MEHEC are preferable. As commercially available examples, STRUCTURE® CEL 12000 M (MEHEC), STRUCTURE® CEL 4400 E (EHEC) and STRUCTURE® CEL 500 M (HM-EHEC) may be mentioned (available from AkzoNobel, the number indicating the viscosity in cps).

### Anionic Thickening Polymer

Any cosmetically acceptable anionic thickening polymers, which are usually employed in the field of hair cosmetic compositions, may be used as the polymeric thickening agent in the composition of the present invention. The composition may include a single kind or multiple kinds of anionic polymer, and may further include one or more non-ionic, cationic and/or amphoteric polymers in combination with the anionic polymer.

Examples for the anionic polymer include anionic polysaccharides and derivatives thereof, such as alginate, pectin, hyaluronate, anionic gums such as xanthan gum, dehydroxanthan gum, hydroxypropyl xanthan gum, gum arabic, gum karaya or gum tragacanth, or anionic cellulose derivatives such as carboxymethyl cellulose (CMC).

Further examples include synthetic anionic polymers such as polyacrylic acid or copolymers containing acrylic acid in combination with neutral vinylic and/or acrylic monomers, and salts thereof such as sodium polyacrylate.

Xanthan gum, hydroxypropyl xanthan gum and dehydroxanthan gum are particularly preferable in view of pH stability.

### Cationic Thickening Polymer

Any cosmetically acceptable cationic thickening polymers, which are usually employed in the field of hair cosmetic compositions, may be used as the polymeric thickening agent in the composition of the present invention. The composition may include a single kind or multiple kinds of cationic polymer, and may further include one or more non-ionic, anionic and/or amphoteric polymers in combination with the cationic polymer.

A cationic polymer is a polymer having a cationic group or a group capable of being ionized into a cationic group. The cationic polymer typically is a polymer containing an amino group or an ammonium group in a side chain of the polymer chain, or a polymer including a diallyl quaternary ammonium salt as a constituent unit, and examples thereof include cationized cellulose, cationic starch, cationic guar gum, a vinylic or (meth)acrylic polymer or copolymer having quaternary ammonium side chains, a polymer or copolymer of a diallyl quaternary ammonium salt, and quaternized polyvinylpyrrolidone.

As an example of the vinylic or (meth)acrylic polymer or copolymer having quaternary ammonium side chains, poly(2-methacryloxyethyltrimethylammonium chloride) (Polyquaternium-37) may be mentioned.

Specific examples of the quaternized polyvinylpyrrolidone include quaternary ammonium salts synthesized from a copolymer of vinylpyrrolidone (VP) and dimethylaminoethyl methacrylate, and diethyl sulfate (polyquaternium 11, for example, GAFQUAT 734, GAFQUAT 755 and GAFQUAT 755N (all by ISP Japan, Ltd.)).

Specific examples of the cationized cellulose include a polymer of a quaternary ammonium salt obtained by adding glycidyltrimethylammonium chloride to hydroxyethylcellulose (polyquaternium-10, for example, RHEOGUARD G and RHEOGUARD GP (all by Lion Corp.), POLYMER JR-125, POLYMER JR-400, POLYMER JR-30M, POLYMER LR-400 and POLYMER LR-30M (all by Amerchol Corp.)), and a hydroxyethylcellulose/dimethyldiallylammonium chloride copolymer (polyquaternium-4, for example, CELQUAT H-100, CELQUAT L-200 (all by National Starch and Chemical Company)), and a polymeric quaternary ammonium salt of hydroxyethyl cellulose reacted with a trimethyl ammonium substituted epoxide and a lauryl dimethyl ammonium substituted epoxide (polyqauternium-67, for example, SoftCat Polymer SX-1300H).

Among these examples, Polyquaternium-37 is particularly preferred.

### Amphoteric Thickening Polymer

The amphoteric polymer contains both, cationic and anionic groups. From a structural point of view, the amphoteric polymer may be derived from any of the described cationic polymer types by the additional introduction of anionic groups or comonomers.

Like the cationic polymers, the amphoteric polymers may also provide a hair-conditioning effect, specifically in case of a positive net charge.

Any cosmetically acceptable amphoteric thickening polymers, which are usually employed in the field of hair cosmetic compositions, may be used as the polymeric thickening agent in the composition of the present invention. The composition may include a single kind or multiple kinds of amphoteric polymer, and may further include one or more non-ionic, anionic and/or cationic polymers in combination with the amphoteric polymer.

As examples for the amphoteric polymer, carboxyl-modified or sulfonate-modified cationic polysaccharides such as carboxymethylchitosan may be mentioned.

Further examples include copolymers of cationic vinylic or (meth)acrylic monomers with (meth)acrylic acid, such as dimethyldiallylammonium chloride/acrylic acid copolymer (polyquaternium-22, for example, MERQUAT 280, MERQUAT 295; Nalco Company).

As an amphoteric polymer, Polyquaternium-22 is particularly preferred.

### Surfactants

The composition may comprise one or more surfactants. As surfactant, any of a cationic surfactant, a nonionic surfactant, an amphoteric surfactant and an anionic surfactant can be used. It is also possible to use two or more types of surfactants in combination.

The cationic surfactant may be mono or di or tri long alkyl chain quaternary ammonium salt, having one to three C₈-C₂₄ alkyl residues and one to three C₁-C₄ alkyl residues. Preferred are mono and di long alkyl chain comprising cationic surfactants and more preferred are mono long alkyl chain comprising cationic surfactants

Preferably the cationic surfactant is selected from the compounds with the general formula 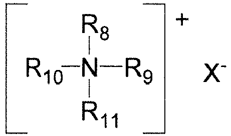
wherein R₈ is a saturated or unsaturated, branched or straight alkyl chain with 8-22 C atoms or

   R₁₂-CO-NH-(CH₂)ₙ-
wherein R₁₂ is a saturated or unsaturated, branched or straight alkyl chain with 7-21 C atoms and n is an integer of 1 - 4, or

   R₁₂-CO-O-(CH₂)ₙ-
wherein R₁₂ is a saturated or unsaturated, branched or straight alkyl chains with 7-21 C atoms and n is an integer of 1 - 4, and
wherein R₉ and R₁₀ are saturated or unsaturated, branched or straight alkyl chain with 1-22 C atoms or

   R₁₂-CO-NH-(CH₂)ₙ-

   or

   R₁₂-CO-O-(CH₂)ₙ-
wherein R₁₂ and n has the same meaning as above, and
R₁₁ is an alkyl group with 1 to 4 carbon atoms, hydroxyl alky chain with 1 to 4 carbon atoms, or ethoxy or propoxy group with a number of ethoxy or propoxy groups varying in the range of 1 to 4, and X is chloride, bromide, methosulfate or ethosulfate. Examples for cationic surfactants include long-chain quaternary ammonium compounds which can be used alone or in admixture with one another, such as cetyl trimethyl ammonium chloride, myristyl trimethyl ammonium chloride, behentrimonium chloride, trimethyl cetyl ammonium bromide, stearyl trimethyl ammonium chloride, stearyl trimonium chloride and stearamidopropyltrimonium chloride.

Examples of the nonionic surfactant include polyoxy-C₁₋₄-alkylene C₈₋₂₄-alkyl ether, polyoxy-C₁₋₄-alkylene C₈₋₂₄-alkylene alkenyl ether, higher (C₁₂-C₂₄) fatty acid sucrose ester, polyglycerin C₈₋₂₄-fafty acid ester, higher (C₁₂-C₂₄) fatty acid mono- or diethanolamide, polyoxyethylene hardened castor oil, polyoxyethylene sorbitan C₈₋₂₄-fatty acid ester, polyoxyethylene sorbit C₈₋₂₄-fatty acid ester, C₈₋₂₄-alkyl saccharide surfactant, C₈₋₂₄-alkylamine oxide, and C₈₋₂₄-alkylamidoamine oxide.

Examples of the amphoteric surfactant include an imidazoline-based surfactant, a carbobetaine-based surfactant, an amidobetaine-based surfactant, a sulfobetaine-based surfactant, a hydroxysulfobetaine-based surfactant and an amidosulfobetaine-based surfactant.

Examples of the anionic surfactant include alkylbenzenesulfonate, alkyl or alkenyl ether sulfate, alkyl or alkenyl sulfate, olefin sulfonate, alkanesulfonate, saturated or unsaturated fatty acid salts, alkyl or alkenyl ether carboxylate, α-sulfo fatty acid salts, N-acylamino acid type surfactants, phosphoric acid mono- or diester type surfactants, and sulfosuccinate. Examples of the alkyl ether sulfate include polyoxyethylene alkyl ether sulfate. Examples of the counterion for the anionic residues of these surfactants include an alkalimetal ion such as sodium ion or potassium ion; an alkaline earth metal ion such as calcium ion or magnesium ion; an ammonium ion; and an alkanolamine having 1 to 3 alkanol groups each having 2 or 3 carbon atoms (for example, monoethanolamine, diethanolamine, triethanolamine, or triisopropanolamine).

The surfactant can be used singly or in combination of two or more kinds. The total surfactant concentration in the compositions is in the range of 0.05 to 10%, preferably 0.1 to 7.5%, and more preferably 0.2 to 5%, by weight calculated to the total of the composition.

### Film-forming polymers

The composition according to the present invention may comprise one or more film-forming polymers, preferably selected from anionic, nonionic, amphoteric and cationic polymers.

Examples for non-ionic polymer include first of all vinylpyrrolidon polymers either homopolymers or copolymers with, especially, vinylacetate. Those are known with the trade name "Luviskol" as homopolymers Luviskol K 30, K 60 or K 90 as well as copolymers Luviskol VA 55, VA 64, Plus from BASF AG and advantage LS-E from ISP.

As amphoteric polymers which can be used alone or in mixture with at least one additional nonionic polymer, reference is here made in particular to copolymers of N-octyl acrylamide, (meth)acrylic acid and tert.-butyl aminoethyl methacrylate of the type "Amphomer®"; copolymers from methacryloyl ethyl betaine and alkyl methacrylates of the type "Yukaformer®", e.g., the butyl methacrylate copolymer "Yukaformer® Am75"; copolymers from monomers containing carboxyl groups and sulfonic groups, e.g., (meth)acrylic acid and itaconic acid, with monomers such as mono- or dialkyl aminoalkyl (meth)acrylates or mono- or dialkyl aminoalkyl (meth)-acrylamides containing basic groups, in particular amino groups; copolymers from N-octyl acrylamide, methyl methacrylate, hydroxypropyl methacrylate, N-tert.-butyl aminoethyl methacrylate and acrylic acid, as well as the copolymers known from US-A 3,927,199.

Examples for anionic polymers, which can be used alone or in combination with non-ionic polymers, include vinyl alkyl ether, in particular methyl vinyl ether/maleic acid copolymers, obtained by hydrolysis of vinyl ether/maleic anhydride copolymers, distributed under the trade name "Gantrez® AN or ES". These polymers may also be partly esterified, as for example, "Gantrez® ES 225" or "ES 435", the ethyl ester of an ethyl vinyl ether/maleic acid copolymer, or the butyl or isobutyl ester thereof.

Further useful anionic polymers are in particular vinyl acetate/crotonic acid or vinyl acetate/vinyl neodecanoate/crotonic acid copolymers of the type "Resyn®"; sodium acrylate/vinyl alcohol copolymers of the type "Hydagen® F", sodium polystyrene sulfonate, e.g., "Flexan® 130"; ethyl acrylate/acrylic acid/N-tert.-butyl acrylamide copolymers of the type "Ultrahold®"; vinyl pyrrolidone/vinyl acetate/itaconic acid copolymers, acrylic acid/acrylamide copolymers or the sodium salts thereof of the type "Reten®"; etc.

Further examples for anionic polymers are acrylate copolymers available under trade name Salcare SC 81, PEG/PPG 25/25 dimethicone / acrylate copolymer available under the trade name Luviglex Silk from BASF, Acrylates/t-butylacrylamide copolymer available under the trade name Ultrahold Strong, Advantage LC-E which is vinylcaprolactam/PVP/dimethylaminoethylmethacrylate copolymer and VA/crotonates copolymer available under the trade name Luviset CA 66.

Examples for cationic polymers include Polyquaternium 11, Polyquaternium 16, Polyquaternium 22 and Polyquaternium 28, Polyquaternium 30, Polyquaternium 37, Polyquaternium 36, Polyquaternium 46, Polyquaternium 24, Polyquaternium 67, and Polyquaternium 72.

The concentration of the one or more film-forming polymer is in the range of 0.1 to 15%, preferably 0.2 to 10%, more preferably 0.25 to 7.5% and most preferably 0.5 to 7.5% by weight, calculated to the total of the composition.

### Solvents

The compositions of the present invention may be aqueous, aqueous-alcoholic (ethanolic) and alcoholic (ethanolic). In case the compositions are aqueous-alcoholic, the water concentration may vary in the range of 1 to 94%, preferably 2 to 80%, more preferably 5 to 75% and most preferably 10 to 60% by weight, calculated to the total of the composition.

The compositions may also comprise an additional organic solvent other than ethanol. Suitable examples include propanol, isopropanol, benzyl alcohol, benzyloxyethanol, alkylene carbonates such as ethylene carbonate and propylene carbonate, phenoxyethanol, butanol, isobutanol, cyclohexane, cyclohexanol, hexyleneglycol, ethylenecarbonate, ethyleneglycol monoethylether, ethylene glycol monobutyl ether, ethylene glycol monophenyl ether, 1-phenylethylalcohol, 2-phenylethylalcohol, o-methoxyphenol. The concentration of organic solvent can be in the range of 1 to 40%, preferably 1 to 25% by weight, calculated to the total composition.

### Propellant

The composition of the present invention may be provided in a pressurised container equipped with an activator and a releasing nozzle. Pressurized compositions comprise one or more propellants which may be filled into one or two chamber aerosol cans. Suitable propellants are lower alkanes such as n-butane, i-butane, propane, butane, liquid nitrogen, pressurised air, carbon dioxide, hydrogen, oxygen and LPG. The pressure in the can is typically less than 12 bar, preferably around 8.5 bar. The concentration of one or more propellants is in the range of 1 to 30%, preferably 2 to 20% more preferably 5 to 15% by weight calculated to the total composition.

### UV filter

The compositions may further comprise one or more UV filters which may be watersoluble or hydrophobic oil-soluble UV filters or mixtures thereof for protection of the hair from damaging effects of sun light. The UV-absorbing substance is preferably selected from the following compounds: 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2.4-dihydroxybenzophenone, 2.2'.4.4'-tetrahydroxy- benzophenone, 2-hydroxy-4-methoxybenzophenone and its 5-sulfonic acid or the sodium salt thereof, 2.2'-dihydroxy-4.4'-dimethoxybenzophenone, 2-hydroxy-5-chlorobenzophenone, 2.2'-dihydroxy-4-methoxybenzophenone, 2.2'-dihydroxy-4.4'-dimethoxy-5.5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof and/or 3-(4'-methyl benzylidene)-DL-campher, polysilicone-15. The preferred amount of the UV-absorber ranges from about 0.01 % to 2.5%, more preferably from 0.05 % to 1 % by weight, calculated to the total composition.

### pH value

The pH of the compositions is in the range of 4 to 9, preferably 4.5 to 8, more preferably 5 to 7.5 and most preferably 5.5 to 7.5. The pH value of the compositions may be adjusted with any known cosmetically acceptable inorganic or organic acids and bases. Examples to the organic acids are citric acid, lactic acid, malic acid, gluconic acid, glyoxylic acid, examples for inorganic acids are hydrochloric acid, sulphuric acid. Examples for the bases include sodium hydroxide, ammonia, alkyl or alkanol amines such as monoethanolamine and aminomethylpropanol.

### Other substances

The compositions according to present invention may comprise one or more additional compounds such as silicones, waxes, dyestuffs, fragrances, preservatives, and any other compounds found customarily in cosmetic compositions, especially in hair cosmetic compositions.

### Silicones

The compositions may comprise one or more silicone compounds other than the silicone copolymer. Examples of the silicone include dimethylpolysiloxane, and modified silicone (for example, amino-modified silicone, fluorine-modified silicone, alcohol-modified silicone, polyether-modified silicone, epoxy-modified silicone, or alkyl-modified silicone), but dimethylpolysiloxane, polyether-modified silicone and amino-modified silicone are preferred.

The dimethylpolysiloxane may be any cyclic or non-cyclic dimethylsiloxane polymer, and examples thereof include SH200 series, BY22-019, BY22-020, BY11-026, B22-029, BY22-034, BY22-050A, BY22-055, BY22-060, BY22-083, FZ-4188 (all by Dow Corning Toray Co., Ltd.), KF-9008, KM-900 series, MK-15H, and MK-88 (all by Shin-Etsu Chemical Co., Ltd.).

The polyether-modified silicone may be any silicone having a polyoxyalkylene group, and the group constituting the polyoxyalkylene group may be an oxyethylene group or an oxypropylene group. More specific examples include KF-6015, KF-945A, KF-6005, KF-6009, KF-6013, KF-6019, KF-6029, KF-6017, KF-6043, KF-353A, KF-354A, KF-355A (all by Shin-Etsu Chemical Co., Ltd.), FZ-2404, SS-2805, FZ-2411, FZ-2412, SH3771M, SH3772M, SH3773M, SH3775M, SH3749, SS-280X series, BY22-008 M, BY11-030, and BY25-337 (all by Dow Corning Toray Co., Ltd.).

The amino-modified silicone may be any silicone having an amino group or an ammonium group, and examples thereof include an amino-modified silicone oil having all or a part of the terminal hydroxyl groups capped with a methyl group or the like, and an amodimethicone which does not have the terminals capped. A preferred example of the amino-modified silicone may be a compound represented by the following formula: 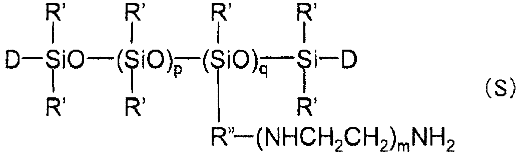 wherein R' represents a hydroxyl group, a hydrogen atom or R^{x}; R^{x} represents a substituted or unsubstituted monovalent hydrocarbon group having 1 to 20 carbon atoms; D represents R^{x}, R"-(NHCH₂CH₂)ₘNH₂, OR^{x}, or a hydroxyl group; R" represents a divalent hydrocarbon group having 1 to 8 carbon atoms; m represents a number from 0 to 3; p and q represent numbers, the sum of which is, as a number average, equal to or greater than 10 and less than 20,000, preferably equal to or greater than 20 and less than 3000, more preferably equal to or greater than 30 and less than 1000, and even more preferably equal to or greater than 40 and less than 800.

Specific examples of suitable commercially available products of the amino-modified silicone include amino-modified silicone oils such as SF8452C, SS-3551 (all by Dow Corning Toray Co., Ltd.), KF-8004, KF-867S, and KF-8015 (all by Shin-Etsu Chemical Co. , Ltd.); and amodimethicone emulsions such as SM8704C, SM8904, BY22-079, FZ-4671, and FZ-4672 (all by Dow corning Toray Co., Ltd.).

The total content of these silicones other than the silicone copolymer is in the range of 0.01 to 5%, by weight, calculated to the total of the composition.

### Waxes

The compositions may comprise optionally one or more waxes. Suitable non-limiting and preferred examples are petrolatum, ozokerit, carnauba wax, paraffin, lanolin wax, candelila wax, bees wax, microcrystalline wax and cocoglycerides. The total content of the waxes is in the range of 0.01 to 2.5%, by weight, calculated to the total of the composition.

### Natural and/or synthetic oil

The compositions may comprise optionally one or more natural or synthetic oil. Natural oils are in principal any triglyceride suitable for cosmetic use. Non-limiting examples are avocado oil, coconut oil, palm oil, sesame oil, peanut oil, whale oil, sunflower oil, almond oil, peach kernel oil, wheat germ oil, macadamia nut oil, night primrose oil, jojoba oil, castor oil, or also olive oil, soy bean oil, and the derivatives thereof. Mineral oils such as paraffin oil and petrolatum are suitably contained within the scope of the present invention. It should as well be noted that compositions of the present invention can contain mixtures of one or more natural oils and mineral oil.

Further, suitable synthetic oil components are in particular esters of fatty alcohols and/or fatty acids, such as isopropyl myristate, palmitate, stearate and isostearate, oleyl oleate, isocetyl stearate, hexyl laurate, dibutyl adipate, dioctyl adipate, myristyl myristate, oleyl erucate, polyethylene glycol and polyglyceryl fatty acid esters, cetyl - palmitate, etc.

The total concentration of such natural and synthetic oils is in the range of 0.01 to 2.5% by weight calculated to the total of the composition.

### Hair direct dyes

In a suitable application form, the compositions may comprise one or more direct hair dyes. Respective examples thereof include cationic, anionic and neutral dyes and their mixtures.

Non-limiting examples for the cationic dyes include Basic Blue 6, Basic Blue 7, Basic Blue 9, Basic Blue 26, Basic Blue 41, Basic Blue 99, Basic Brown 4, Basic Brown 16, Basic Brown 17, Basic Orange 31, Natural Brown 7, Basic Green 1, Basic Red 2, Basic Red 12, Basic Red 22, Basic Red 51, Basic Red 76, Basic Violet 1, Basic Violet 2, Basic Violet 3, Basic Violet 10, Basic Violet 14, Basic Yellow 57 and Basic Yellow 87.

Non-limiting examples for the anionic dyes include Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium or potassium.

Examples for the neutral dyes include nitro dyes. Non-limiting examples thereof are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Plant dyestuffs may also be used as hair colorant within the meaning of the present invention. Examples include henna (red or black), alkanna root, laccaic acid, indigo, logwood powder, madder root and rhubarb powder, etc.

It should be noted that the above dyestuffs are also suitable for use in mixture. When using direct dyes of various categories, their compatibility must be checked.
The above mentioned dyestuffs are also used, especially the anionic ones, for product coloring at reduced concentrations.

The concentration of direct dyes in the compositions of the present invention is usually within the range of 0.001 to 5%, preferably 0.01 to 3% and more preferably 0.05 to 2%, and most preferably 0.1 to 1% by weight, calculated to the total of the composition.

### Formulation of the Composition

The formulation type of the composition is not particularly limited. Preferably, the composition can be provided as a hair styling composition or a leave-on type hair conditioner. Examples of preferred formulations include a pump spray, an aerosol spray, a pump foam, an aerosol foam, a gel, a lotion, a mist and a cream.

Alternately, the composition is provided as a direct dye composition by incorporating at least one direct dye as described above. For formulating the direct dye composition, conventionally known methods may be used.

### Examples

The following examples are to illustrate the invention but not to limit it.

### Example 1 (Reference Example)

### Determination of shine increase:

Four hair tresses were treated four times with a permanent shaping composition available under the brand Goldwell Topform in order to prepare damaged tresses. The tresses were washed with 5% sodium lauryl ether sulphate solutions (pH approximately 5.5) and combed and dried at ambient temperature. Afterwards the tresses were soaked into a 0.05% by weight silicone copolymer (polysilicone-28) comprising composition for 1 min and taken out from the solution and towel dried to approximately 20%, by weight, solution level and dried with a hair drier.

Shine measurements were carried out with a system comprising a polarization camera, a polarized illumination and a cylindrical mount for the tresses. Illuminated light was measured under ambient conditions before and after treating the tresses with the inventive composition. The following results were obtained according to Bossa Nova and Reich Robins methods which are the methods for calculating shine according to two different algorithms. The method is known from a scientific publication by N.Lefaudeux et al., in Journal of Cosmetic Science, 60(2), 153 - 169, 2009.

| | **Bossa Nova** | | | **Reich Robbins** | | |
|---|---|---|---|---|---|---|
| Tress | before | after | % | before | after | % |
| 1 | **17,74** | **18,49** | **+2,23** | **44,49** | **55,66** | **+27,4** |
| 2 | **15,54** | **19,19** | **+23,5** | **42,24** | **47,61** | **+12,7** |
| 3 | **15,62** | **20,94** | **+34,1** | **38,58** | **44,04** | **+14,2** |
| 4 | **12,97** | **14,26** | **+9,95** | **27,89** | **31,26** | **+12,3** |

As obvious from the above results, all tresses had more shine after treatment with the inventive composition.

### Example 2 (Reference Example)

### Determination of heat protection

Heat protection effect of the silicone copolymer was demonstrated with stress strain measurement. Therefore, human hair streaks were bleached with a commercially available bleaching composition and dried at room temperature. The streaks thus obtained were treated with the silicone copolymer solution comprising 1% by weight of the the silicone copolymer for 1 min and dried at room temperature and treated 10 times with a flat iron having a surface temperature of 230°C and shampooed followed by drying. The silicone copolymer and heat treatment cycles were repeated 5 times. Afterwards, forces for upper limit elasticity and breaking were measured. The following results were obtained.

| | Force for upper limit elasticity | Breaking force |
|---|---|---|
| Control | 3.99 N | 6.52 N |
| Inventive | 4.63 N | 7.47 N |

Since for both forces measured, the inventive tress had resulted in a higher force than that of the control streak, the copolymer comprised in the inventive compositions protects the hair against heat.

### Example 3

### Determination of wash fastness

The following compositions were used for wash fastness tests:

| | % by weight | | | |
|---|---|---|---|---|
| Composition | 1 | 2 | 3 | 4 |
| Amphomer HC | 4.0 | 4.0 | 4.0 | 4.0 |
| VP/VA 37 | 12.0 | 12.0 | 12.0 | 12.0 |
| Polysilicone-28 | - | 0.005 | 0.05 | 0.5 |
| Water | to 100 | | | |

Human hair streaks were bleached with a commercially available bleaching composition and dried at room temperature. The streak thus obtained were coloured with a commercially available hair dye under the brand name Topchic shade 6RK. The coloured hair streaks were soaked in a control solution (water), the above described composition 1 which does not comprise the silicone copolymer, and the above-described compositions 2-4 comprising the silicone copolymer.

All streaks were then subjected to a wash test. For this purpose, the streaks were soaked into a shampoo (commercially available shampoo under the brand name Goldwell Dual Senses for coloured hair) solution, 5% by weight, and shaken at a speed of 80 rpm in a water bath having a temperature of 40°C for 20 min. Afterwards the streaks were taken out from the shampoo solution, rinsed off and dried. The L, a and b values were measured before and after the wash test and from the values for each streak a color difference value (ΔE) was calculated. The results are given in the table below.

| | ΔE value |
|---|---|
| Blank (water) | 4.7 |
| Composition 1 | 4.9 |
| Composition 2 | 3.1 |
| Composition 3 | 2.6 |
| Composition 4 | 3.0 |

From the above results, it is clear that the least colour differences, smaller ΔE values, are observed with the streaks treated with the inventive compositions 2-4 comprising Polysilicone-28.

### Example 4 (Reference Example)

### Reduced polymer surface friction

A surface friction measurement was carried out with a method wherein a metal sled pulled over the polymer film and the kinetic force required therefore is measured in mN. On a glass plate, polymer films having a thickness of 200 um were prepared from the compositions 1-4 of Example 3 and the films were dried. Subsequently, kinetic force was measured for each film at least four times using the Zwick Roell device. The equipment was operated according to its usage instructions included in the manual. The following results were obtained.

| | Kinetic force (mN) |
|---|---|
| Composition 1 | 370 |
| Composition 2 | 245 |
| Composition 3 | 180 |
| Composition 4 | 80 |

From the above results it is beyond any doubt that the inclusion of Polysilicone-28 into the polymer film reduced the friction force. It is also dependent on the concentration of the silicone copolymer. For the reference composition 1 which does not include any Polysilicone-28, the largest kinetic force is observed. With an increasing content of Polysilicone-28, the kinetic force is reduced.

### Example 5: Hair spray

| | |
|---|---|
| Polysilicone-28 | 0,05 % |
| Ethanol | Add 100,00 % |

### Example 6: Hair spray

| | |
|---|---|
| VP/VA Copolymer | 12,00 % |
| Acrylates/Octylacrylamide Copolymer | 4,00 % |
| Aminomethyl Propanol | 0,51 % |
| Polysilicone-28 | 0,005 % |
| Ethanol | Add 100,00 % |

### Example 7: Hair spray

| | |
|---|---|
| VP/VA Copolymer | 12,00 % |
| Acrylates/Octylacrylamide Copolymer | 4,00 % |
| Aminomethyl Propanol | 0,51 % |
| Polysilicone-28 | 0,05 % |
| Ethanol | Add 100,00 % |

### Example 8: Hair spray

| | |
|---|---|
| VP/VA Copolymer | 12,00 % |
| Acrylates/Octylacrylamide Copolymer | 4,00 % |
| Aminomethyl Propanol | 0,51 % |
| Polysilicone-28 | 0,5 % |
| Ethanol | Add 100,00 % |

### Example 9: Thermospray

| | |
|---|---|
| Polysilicone-28 | 1,00% |
| Ethanol | Add 100,00 % |

### Example 10: Hair spray

| | |
|---|---|
| Water | 4,00 % |
| VP/VA Copolymer | 6,00 % |
| Arylates/Octylacrylamide Copolymer | 4,00 % |
| Aminomethyl Propanol | 1,08 % |
| Ethylhexyl Methoxicinnamate | 0,10 % |
| Fragrance | 0,30 % |
| Polysilicone-28 | 0,05 % |
| Ethanol | Add 100,00 % |
| | |
| Aerosolfilling | |
| Bulk | 50,0 % |
| n-Pentane | 10,0 % |
| Dimethylether | 40,0 % |

### Example 11: Blow-dry spray

| | |
|---|---|
| Ethanol | 6,00 % |
| DMDM-Hydantoin | 0,30 % |
| VP/VA Copolymer | 3,00 % |
| Polyquaternium-16 | 1,00 % |
| Polysilicone-28 | 2,00 % |
| Cetrimoniumchloride | 0,50 % |
| Benzophenone-4 | 0,05 % |
| Fragrance | 0,25 % |
| PEG-40 Hydrogenated Castor Oil | 0,25 % |
| Water | Add 100,00 % |

### Example 12: Thermostyling Spray

| | |
|---|---|
| Ethanol | 6,00 % |
| DMDM-Hydantoin | 0,30 % |
| Polysilicone-28 | 6,00 % |
| Cetrimoniumchloride | 0,50 % |
| Benzophenone-4 | 0,05 % |
| Fragrance | 0,25 % |
| PEG-40 Hydrogenated Castor Oil | 0,25 % |
| Water | Add 100,00 % |

### Example 13: Styling Gel

| | |
|---|---|
| DMDM-Hydantoin | 0,30 % |
| VP/VA Copolymer | 3,00 % |
| Polysilicone-28 | 0,50 % |
| Fragrance | 0,25 % |
| PEG-40 Hydrogenated Castor Oil | 0,25 % |
| Propylenglycol | 0,50 % |
| Sorbitol | 1,00 % |
| Acrylates/Palmeth-25 Acrylate Copolymer | 2,50 % |
| Sodium Hydroxide | 0,43 % |
| Water | Add 100,00 % |

## Claims

1. Use of a composition comprising a silicone graft copolymer comprising an organopolysiloxane segment as a main chain thereof and an unsaturated monomer-derived polymer segment as a side chain thereof,
which is obtainable by firstly reacting an aminopropyl dimethicone with the thiolactone of acetylhomocysteine and then graft copolymerizing the thus obtained mercapto modified dimethicone with a mixture of N,N-dimethylacrylamide and N-t-butylacrylamide, for providing colour retention of artificially coloured hair.

2. The use according to claim 1, wherein the composition is a direct dye composition and comprises at least one direct dye.

3. The use according to claim 1, wherein the composition is applied to the hair after dyeing the hair with a direct dye.

4. The use and the processes according to any one of the claims 1 to 4 wherein the silicone graft copolymer is Polysilicone-28.

5. The use according to any one of the claims 1 to 4, wherein the silicone graft copolymer is comprised in the compositions at a concentration in the range of 0.001 to 10%, preferably 0.005 to 7.5%, more preferably 0.01 to 5% and most preferably 0.05 to 3% by weight calculated to the total of the composition.

6. The use according to any one of the claims 1 to 5, wherein the composition comprises additionally a thickening polymer.

7. The use according to any one of the claims 1 to 6, wherein the composition comprises one or more surfactants.

8. The use according to any one of the claims 1 to 7, wherein the composition comprises one or more film forming polymers, preferably selected from selected from anionic, nonionic, amphoteric and cationic polymers.

9. The use according to any one of the claims 1 to 8, wherein the composition comprises one or more of the following compounds:
- a silicone other than the silicone graft copolymer
- a wax

10. The use according to any one of the claims 1 to 9 wherein the composition comprises one or more organic solvent and/or one or more propellant and/or one or more UV filters.

11. The use according to any one of the claims 1 to 10, wherein the composition comprises water.

12. The use according to any one of the claims 1 to 11, wherein the composition has a pH in the range of 4 to 9.

13. Hair dye composition, comprising the silicone graft copolymer as defined in claim 1 and at least one direct dye.

## Patentansprüche

1. Verwendung einer Zusammensetzung, umfassend ein Siliconpfropfcopolymer, umfassend ein Organopolysiloxansegment als Hauptkette davon und ein von einem ungesättigten Monomer abgeleitetes Polymersegment als eine Seitenkette davon,
erhältlich, indem zunächst ein Aminopropyldimethicon mit dem Thiolacton von Acetylhomocystein umgesetzt wird und dann das so erhaltene mercaptomodifizierte Dimethicon mit einer Mischung von N,N-Dimethylacrylamid und N-t-Butylacrylamid pfropfcopolymerisiert wird,
zum Erhalt von Farbstabilität von künstlich gefärbtem Haar.

2. Verwendung gemäß Anspruch 1, wobei die Zusammensetzung eine Direktfarbstoffzusammensetzung ist und mindestens einen Direktfarbstoff umfasst.

3. Verwendung gemäß Anspruch 1, wobei die Zusammensetzung nach dem Färben des Haars mit einem Direktfarbstoff auf das Haar aufgetragen wird.

4. Verwendung und Verfahren gemäß einem der Ansprüche 1 bis 4, wobei das Siliconpfropfcopolymer Polysilicon-28 ist.

5. Verwendung gemäß einem der Ansprüche 1 bis 4, wobei das Siliconpfropfcopolymer in den Zusammensetzungen zu einer Konzentration im Bereich von 0,001 bis 10 Gew.-%, bevorzugt 0,005 bis 7,5 Gew.-%, mehr bevorzugt 0,01 bis 5 Gew.-% und am meisten bevorzugt 0,05 bis 3 Gew.-%, berechnet auf die Gesamtmenge der Zusammensetzung, enthalten ist.

6. Verwendung gemäß einem der Ansprüche 1 bis 5, wobei die Zusammensetzung zusätzlich ein Verdickungspolymer umfasst.

7. Verwendung gemäß einem der Ansprüche 1 bis 6, wobei die Zusammensetzung ein oder mehrere Tenside umfasst.

8. Verwendung gemäß einem der Ansprüche 1 bis 7, wobei die Zusammensetzung ein oder mehrere filmbildende Polymere umfasst, die bevorzugt aus anionischen, nichtionischen, amphoteren und kationischen Polymeren ausgewählt sind.

9. Verwendung gemäß einem der Ansprüche 1 bis 8, wobei die Zusammensetzung eine oder mehrere der folgenden Verbindungen umfasst:
- ein anderes Silicon als das Siliconpfropfcopolymer
- ein Wachs.

10. Verwendung gemäß einem der Ansprüche 1 bis 9, wobei die Zusammensetzung ein oder mehrere organische Lösungsmittel und/oder ein oder mehrere Treibmittel und/oder einen oder mehrere UV-Filter umfasst.

11. Verwendung gemäß einem der Ansprüche 1 bis 10, wobei die Zusammensetzung Wasser umfasst.

12. Verwendung gemäß einem der Ansprüche 1 bis 11, wobei die Zusammensetzung einen pH-Wert im Bereich von 4 bis 9 aufweist.

13. Haarfärbemittel, umfassend das Siliconpfropfcopolymer, wie in Anspruch 1 definiert, und mindestens einen Direktfarbstoff.

## Revendications

1. Utilisation d'une composition comprenant un copolymère greffé de silicone comprenant un segment organopolysiloxane en tant que chaîne principale de celui-ci et un segment polymère dérivé de monomère insaturé en tant que chaîne latérale de celui-ci,
qui peut être obtenue par mise en réaction dans un premier temps d'une aminopropyl diméthicone avec la thiolactone de l'acétylhomocystéine puis par copolymérisation par greffage de la diméthicone à modification mercapto ainsi obtenue avec un mélange de N,N-diméthylacrylamide et de N-t-butylacrylamide, pour fournir la rétention de couleur des cheveux artificiellement colorés.

2. Utilisation selon la revendication 1, dans laquelle la composition est une composition de colorant direct et comprend au moins un colorant direct.

3. Utilisation selon la revendication 1, dans laquelle la composition est appliquée aux cheveux après coloration des cheveux avec un colorant direct.

4. Utilisation et les procédés selon l'une quelconque des revendications 1 à 4 dans lesquels le copolymère greffé de silicone est le polysilicone-28.

5. Utilisation selon l'une quelconque des revendications 1 à 4, dans laquelle le copolymère greffé de silicone est compris dans les compositions en une concentration dans la plage de 0,001 à 10 %, de préférence de 0,005 à 7,5 %, de manière plus préférée de 0,01 à 5 % et de manière de loin préférée de 0,05 à 3 % en poids calculé par rapport au poids total de la composition.

6. Utilisation selon l'une quelconque des revendications 1 à 5, dans laquelle la composition comprend en outre un polymère épaississant.

7. Utilisation selon l'une quelconque des revendications 1 à 6, dans laquelle la composition comprend un ou plusieurs tensioactifs.

8. Utilisation selon l'une quelconque des revendications 1 à 7, dans laquelle la composition comprend un ou plusieurs polymères filmogènes, de préférence sélectionnés à partir de polymères anioniques, non-ioniques, amphotères et cationiques.

9. Utilisation selon l'une quelconque des revendications 1 à 8, dans laquelle la composition comprend un ou plusieurs des composés suivants :
- une silicone autre que le copolymère greffé de silicone
- une cire.

10. Utilisation selon l'une quelconque des revendications 1 à 9, dans laquelle la composition comprend un ou plusieurs solvants organiques et/ou un ou plusieurs propulsifs et/ou un ou plusieurs filtres UV.

11. Utilisation selon l'une quelconque des revendications 1 à 10, dans laquelle la composition comprend de l'eau.

12. Utilisation selon l'une quelconque des revendications 1 à 11, dans laquelle la composition a un pH dans la plage de 4 à 9.

13. Composition de coloration pour les cheveux, comprenant le copolymère greffé de silicone tel que défini dans la revendication 1 et au moins un colorant direct.
